# EUROPÄISCHE PATENTANMELDUNG

(11) **EP 1 922 986 A1**
(43) Veröffentlichungstag der Anmeldung: **21.05.2008**
(21) Anmeldenummer: 06023706.2
(22) Anmeldetag: 15.11.2006
(51) Int. Cl.: A61B 5/00

(54) **Analysevorrichtung zur Bestimmung eines Analyten in vivo im Körper eines Patienten**

(71) Anmelder: Roche Diagnostics GmbH, 68305 Mannheim (DE); F.HOFFMANN-LA ROCHE AG, 4070 Basel (CH)
(72) Erfinder: Haar, Hans-Peter, 69168 Wiesloch (DE); Rösicke, Bernd, 68305 Mannheim (DE)
(74) Vertreter: Petirsch, Markus

(57) **Zusammenfassung**

Analysevorrichtung zur Bestimmung eines Analyten in vivo im Körper eines Patienten, umfassend eine zum Einführen durch die Hautoberfläche (4) in den Körper ausgebildete Perkutan-Messsonde (2) mit einem Sondenkopf (6), der einen elektrochemischen Analysesensor (7) mit zwei Messelektroden (25,26,27) aufweist, eine am Körper tragbare Sondenanschlusseinheit (3) zum Anschließen an die Perkutan-Messsonde (2), eine mit den Messelektroden (25,26,27) verbundene Messschaltung (22), wobei die Messschaltung (22) bei Kontakt der Messelektroden (25,26,27) mit einer Körperflüssigkeit ein Messsignal erzeugt, das für das gewünschte analytische Resultat charakteristisch ist, eine Auswerteschaltung (23), um Messsignale aus der Messschaltung (22) auszuwerten und eine Information über das gewünschte analytische Resultat zu gewinnen, bei welcher die Messschaltung (22) als Bestandteil einer Sondenkopfelektronik (10) in dem Sondenkopf (6) integriert ist, der Sondenkopf (6) an einen Lichtleiter (8,18) angekoppelt ist, der Sondenkopf (6) einen optischen Sender (17) aufweist, um elektrische Signale der Sondenkopfelektronik (10) in Lichtsignale umzuwandeln und um die Lichtsignale durch den an dem Sondenkopf (6) angekoppelten Lichtleiter (8,18) zu übertragen, und die Sondenanschlusseinheit (3) einen Lichtempfänger (28) enthält, um die Lichtsignale von der Messsonde (2) zur Weiterverarbeitung zu empfangen.

## Beschreibung

Die vorliegende Erfindung betrifft eine Analysevorrichtung zur Bestimmung eines Analyten in vivo im Körper eines Patienten, umfassend eine Perkutan-Messsonde mit einem Sondenkopf, der einen elektrochemischen Analysesensor mit zwei Messelektroden aufweist, eine am Körper tragbare Sondenanschlusseinheit, eine Messschaltung zum Anschließen der Messelektroden und eine Auswerteeinheit. Die Messschaltung erzeugt bei Kontakt der Messelektroden mit der Körperflüssigkeit ein Messsignal, das für das gewünschte analytische Resultat charakteristisch ist. In der Auswerteschaltung werden Messsignale der Messschaltung ausgewertet, um eine Information über das gewünschte analytische Resultat zu gewinnen.

Analysesysteme zur Bestimmung eines Analyten in vivo im Körper eines Patienten werden insbesondere dort eingesetzt, wo eine Analyse einer Körperflüssigkeit, insbesondere von Blut, mehrmals täglich durchgeführt werden muss. Ein typisches Beispiel hierfür ist die Überwachung des Glucosegehalts im Blut bei Diabetikern. Der Glucosegehalt soll möglichst kontinuierlich kontrolliert werden. Die Verwendung von Stechgeräten, um eine kapillare Blutprobe zu erhalten, ist mit Schmerzen verbunden und entsprechend zeitaufwendig. Dies wird von vielen Patienten als unkomfortabel empfunden, so dass sie eine regelmäßige Bestimmung des Analyten im Körper unterlassen. Jedenfalls erfolgt die Überwachung diskontinuierlich, in der Praxis höchstens vier- bis fünfmal am Tag. Gerade bei Diabetikern kann das fehlende kontinuierliche Monitoring und die ungenaue, aufgrund von fehlenden Analyseergebnissen basierende Zugabe von Insulin zu schweren Schäden und Spätfolgen führen.

Eine Vielzahl von Analysesystemen zur kontinuierlichen Überwachung von Analyten, wie beispielsweise von Glucose oder Laktat im Blut bzw. in einer (interstitiellen) Körperflüssigkeit sind im Stand der Technik beschrieben. Hierzu wird ein Analysesensor, der auf elektrochemischen Messprinzipien basiert, in eine Blutbahn oder unter die Hautoberfläche eingeführt. Ein Problem dieser Analysesysteme ist die generell unzureichende Reproduzierbarkeit der gewonnenen analytischen Resultate. Häufig sind derartige Systeme relativ groß und unflexibel und somit entsprechend unkomfortabel. Dies führt zu Beschränkungen in der Beweglichkeit der Patienten.

Aus der US 6,560,471 B1 ist ein Analysesystem bekannt, bei dem ein Teil eines elektrochemischen Analysesensors mit mehreren Messelektroden unter die Hautoberfläche angeordnet wird. Der außerhalb der Haut positionierte Teil des Analysesensors wird an eine Sensorkontrolleinheit angekoppelt, die eine Messschaltung zur Auswertung der von den Messelektroden aufgenommenen Messsignale enthält. Die in der Sensorkontrolleinheit ausgewerteten Messsignale werden drahtlos an eine Anzeigeeinheit übertragen. Die Anzeigeeinheit ist als externes Gerät konzipiert oder lässt sich am Körper tragen bzw. vom Patienten mitführen. Die Sensorkontrolleinheit kann auch über Anzeigen verfügen, um eine Information über das gewünschte analytische Resultat anzuzeigen. Darüber hinaus ist vorgesehen, ein Warnsignal auszugeben, wenn beispielsweise ein gemessener Glucosewert unter einen bestimmten Sollwert fällt. In einer als externes Gerät ausgebildeten Anzeigeeinheit können neben der Anzeige der Analysewerte weitere Auswertungen und eine Archivierung der Messdaten vorgenommen werden.

Einen anderen Weg beschreitet das Analysegerät, dass von R. Beach, F. von Küster, F. Moussy, in "Subminiatur implantable Potentiostat and modified commercial telemetry device for remote glucose monitoring" in IEEE Transactions on instrumentation and measurement, vol. 48, no.6, Dezember 1999, vorgestellt wurde. Das Analysegerät umfasst einen elektrochemischen Glucosesensor, eine als Potentiostat ausgebildete Messschaltung, einen Optokoppler zur Entkopplung der Messschaltung von einer drahtlosen Übertragungseinheit und eine Stromversorgung in Form einer Batterie. Diese Komponenten werden gemeinsam unter die Haut des Patienten implantiert. Das Analysegerät sendet die einem Glucosewert entsprechenden Messergebnisse als elektromagnetische Wellen an eine externe Empfangseinheit, die einen Computer umfassen kann. Das Analysegerät ist sehr groß und hat Dimensionen von 4 cm Länge, 2,6 cm Breite und einer Höhe von 1,8 cm. Seine Implantation erfordert einen aufwendigen chirurgischen Eingriff. Darüber hinaus muss immer dann, wenn die Batterie des Analysegerätes entladen ist, ein weiterer chirurgischer Eingriff vorgenommen werden. Die Lebensdauer der Batterie ist mit maximal einem Jahr angegeben, bei kontinuierlicher Messung wird sie jedoch auf nur drei Monate geschätzt. Damit ist das vorgeschlagene Gerät für den Alltagseinsatz nicht tauglich und mit hohen Kosten für die Entnahme und Einpflanzung in dem Patienten verbunden.

Auf dieser Grundlage befasst sich die Erfindung mit dem Problem, eine Analysevorrichtung mit einem elektrochemischen Sensor zur Bestimmung eines Analyten in vivo im Körper eines Patienten vorzuschlagen, der gegenüber den im Stand der Technik bekannten Analysegeräten verbesserte Eigenschaften, insbesondere im Bezug auf den Tragekomfort und die Messgenauigkeit sowie Zuverlässigkeit, aufweist.

Gelöst wird dieses Problem durch eine Analysevorrichtung mit den Merkmalen des Anspruchs 1.

Die erfindungsgemäße Analysevorrichtung zur Bestimmung eines Analyten in vivo im Körper eines Patienten umfasst eine Perkutan-Messsonde zum Einführen durch die Hautoberfläche in den Körper. Die Sonde hat einen Sondenkopf, der einen elektrochemischen Analysesensor mit mindestens zwei Messelektroden und einen optischen Sender aufweist, um elektrische Signale einer Sondenkopfelektronik in Lichtsignale umzuwandeln und um die Lichtsignale durch einen an dem Sondenkopf angekoppelten Lichtleiter zu übertragen. Eine Messschaltung zum Anschließen der Messelektroden ist als Bestandteil der Sondenkopfelektronik in dem Sondenkopf integriert. Bei Kontakt der Messelektroden mit einer Körperflüssigkeit erzeugt die Messschaltung ein Messsignal, dass für das gewünschte analytische Resultat charakteristisch ist. Eine am Körper tragbare Sondenanschlusseinheit dient zum Anschließen an die Perkutan-Messsonde. Die Sondenanschlusseinheit enthält einen Lichtempfänger, um die Lichtsignale von der Messsonde zur Weiterverarbeitung zu empfangen. Die Analysevorrichtung umfasst weiter eine Auswerteschaltung, um Messsignale der Messschaltung auszuwerten und eine Information über das gewünschte analytische Resultat zu gewinnen.

Die im Stand der Technik bekannten Analysevorrichtungen sehen elektrische Leitungen zwischen den Messelektroden und der Messschaltung vor. Im Rahmen der Erfindung wurde festgestellt, dass diese elektrischen Leitungen zwischen den Messelektroden und einer über der Hautoberfläche lokalisierten Messschaltung nicht nur als störend in Bezug auf die Bewegungsfreiheit des Patienten empfunden werden. Vielmehr führt die Verwendung der relativ langen elektrischen Leitungen auch zu Problemen bei der Signalübertragung. Bei den eingesetzten elektrochemischen Analysesensoren mit zwei oder auch drei Messelektroden fließen Ströme im Bereich vom Piko-Ampere. Die Impedanz der Messanordnung liegen im Bereich von mehreren hundert Megaohm. Hieraus ergeben sich sehr hohe Anforderungen an die Isolierung der Signalleitungen und an eine mögliche Steckverbindung zwischen den Leitungen und der in der Regel außerhalb des Körpers angeordneten Messschaltung. Es lässt sich somit praktisch nicht ausschließen, dass Kriechströme auftreten, die die Messergebnisse verfälschen. Diese Probleme werden bei einer Messanordnung mit drei Elektroden noch verstärkt. Hinzu kommt, dass aufgrund der langen Leitungen elektromagnetische Störungen von außen in die Messanordnung einkoppeln können, die Störspannungen und Störströme hervorrufen und so die Messergebnisse beeinflussen.

Die vorliegende Erfindung beschreitet einen gänzlich anderen Weg um lange elektrische Leitungen für die Übertragung der Messsignale vom Analysesensor zur Messschaltung zu vermeiden. Die Messschaltung wird als Bestandteil der Sondenkopfelektronik in die Messsonde integriert. Das von der Messschaltung erzeugte elektrische Messsignal wird von einem im Sondenkopf angeordneten elektrooptischen Wandler in Lichtsignale umgewandelt. Die Lichtsignale werden dann über Lichtleiter von der unter der Hautoberfläche positionierten Messsonde an die Sondenanschlusseinheit, die außerhalb des Körpers am Körper tragbar ist, übertragen. Die Übertragung des Messsignals durch die Hautoberfläche hindurch erfolgt also als Lichtsignal, das in der Sondenanschlusseinheit von einem optischen Lichtempfänger empfangen und in ein elektrisches Signal umgewandelt wird, so dass es weiter verarbeitet werden kann. Auf diese Weise werden die erläuterten Probleme bei Zuleitungen überwunden. Die optisch übertragenen Signale sind frei von den oben genannten Beeinflussungen. Insbesondere können Störeinflüsse von außen, die auf längere parallel verlaufene elektrische Leitungen einwirken können, und die mit der elektrischen Verbindung der Leitungen mit der Messschaltung und der Sondenanschlusseinheit einhergehenden Probleme überwunden werden.

Bei der Übertragung von Daten in Form von optischen Lichtsignalen von der Messsonde an die Sondenanschlusseinheit bestehen prinzipiell zwei unterschiedliche Vorgehensweisen:
a) Die von der Messschaltung erzeugten Messsignale, die für das analytische Resultat charakteristisch sind, werden in Form von optischen Lichtsignalen über die Lichtleiter übertragen. In diesem Fall werden die "Rohdaten" der Messanordnung übertragen. Die übertragenen Daten entsprechen einem Strom- oder Widerstandsmesswert, aus dem in der Auswerteeinheit das analytische Resultat ermittelt werden kann.
b) Die Sondenkopfelektronik der Messsonde umfasst bevorzugt neben der Messschaltung auch Teile der Auswerteschaltung. Das von der Messschaltung erzeugte Messsignal wird an die Auswerteschaltung übergeben und weiterverarbeitet, so dass aus den "Rohdaten" Zwischenergebnisse erzeugt werden, die in optische Signale umgewandelt und an die Sondenanschlusseinheit übertragen werden. In der Sondenanschlusseinheit ist ein weiterer Teil der Auswerteschaltung integriert, so dass die Zwischenergebnisse verarbeitet werden, um das analytische Resultat zu ermitteln.
   Es kann auch die komplette Auswerteschaltung in der Sondenkopfelektronik integriert sein.

In der Sondenanschlusseinheit wird das empfangene Lichtsignal in ein elektrisches Signal rückgewandelt, das entweder direkt zur Anzeige gebracht oder beispielsweise über eine Funkverbindung an eine externe Anzeigeeinheit übertragen wird.

Vorteilhafterweise werden digitale Signale über den Lichtleiter übertragen. Dazu ist im Sondenkopf der Perkutan-Messsonde ein Analog-Digital-Wandler (A/D-Wandler) integriert, der die elektrischen Signale der Sondenkopfelektronik in digitale Signale umwandelt. Auf diese Weise können von dem optischen Sender im Sondenkopf digitale Lichtsignale von der Messsonde zu der Sondenanschlusseinheit transportiert werden. Eine Übertragung digitaler Signale ist weit weniger störanfällig als die Übertragung analoger optischer Signale. Die weitere Verarbeitung in der Sondenanschlusseinheit ist auf einfache, bekannte Weise möglich.

Der Begriff "Perkutan-Messsonde" wird derart verstanden, dann die Messsonde durch die Hautoberfläche in den Körper eingeführt wird. Die Sonde befindet sich damit in der Haut, also in einer der mehreren Schichten der Haut (cutis). Im Sinne der Erfindung sind aber auch Messsonden mit umfasst, die in dem Sinne durch die Haut eingeführt werden, dass sie unterhalb der Haut, also subkutan, angeordnet sind. Jedenfalls ist die Perkutan-Messsonde unterhalb der Hautoberfläche im Körper angeordnet.

Bevorzugt ist die Messschaltung eine Potentiostatschaltung, also eine Schaltungsanordnung, die die Funktion eines Potentiostaten ausführt, durch die die Spannung zwischen den beiden Messelektroden (Arbeitselektrode, Gegenelektrode) eingestellt wird. Die Einstellung kann auf einem vorgegebenen Wert stattfinden oder sie kann eingeregelt werden.

Messsonden mit Potentiostatschaltungen sind besonders für die Langzeitmessung von Analyten in Körperflüssigkeiten geeignet. Insbesondere wird eine kontinuierliche oder quasi-kontinuierliche Messung von Glucose im Körper durchgeführt. Der in dem Sondenkopf der Perkutan-Messsonde angeordnete elektrochemische Analysesensor hat bei Langzeitmessungen bevorzugt drei Messelektroden, nämlich eine Arbeitselektrode, eine Gegenelektrode und eine zusätzliche Referenzelektrode. Mit dieser Anordnung lässt sich eine sehr exakte Messung durchführen. Dabei wird analog zur chemischen Umsetzung zwischen den Messelektroden ein sehr kleiner elektrischer Strom erzeugt, der über die Arbeits- und Gegenelektrode zur Messschaltung fließt. Die Messgenauigkeit ist davon abhängig, dass die Spannung zwischen der Arbeits- und Gegenelektrode in der Messsonde genau kontrolliert wird. Um dies unabhängig von Störeinflüssen zu gewährleisten wird mit der Potentiostatschaltung die Spannung zwischen der Arbeitselektrode und einer Referenzelektrode hochohmig gemessen. Diese Spannung wird mit einer zwischen der Arbeitselektrode und Gegenelektrode gewünschten Sollspannung verglichen. Die Potentiostatschaltung regelt den Strom durch die Gegenelektrode so ein, dass die Abweichung der gemessenen Spannung (Istwert) zwischen Referenzelektrode und Arbeitselektrode und der Spannung (Sollwert) zwischen Arbeitselektrode und Gegenelektrode verschwindet, also zu Null wird. Somit wird sichergestellt, dass zwischen der Arbeitselektrode und der Gegenelektrode das gewünschte Potential anliegt. Der dann im Stromkreis durch Arbeitselektrode und Gegenelektrode gemessene Strom ist ein Maß für das gewünschte analytische Resultat, insbesondere bei der Bestimmung von Glucose ein Maß für den Glucosegehalt im Blut.

Die Verwendung der Referenzelektrode führt zum einen zu einer sehr exakten Messung. Zum anderen führt ihre hohe Impedanz von mehreren 100 Megaohm jedoch zu den damit einhergehenden, oben beschriebenen Problemen. Gerade in Kombination mit den sehr geringen Strömen im Piko-Ampere-Bereich kann es zu Verfälschungen des Messergebnisses kommen, wenn die Elektroden des elektrochemischen Analysesensors weit von der Potentiostatschaltung entfernt sind. Darüber hinaus können Verfälschungen durch die elektrischen galvanischen Kontakte hervorgerufen werden, da zwischen den nicht isolierten Elektroden und dem Ausgang bzw. den Versorgungssteckern parasitäre Oberflächen-Widerstände durch Schweiß auftreten können. Dies beides wird erfindungsgemäß dadurch verhindert, dass der elektrochemische Analysesensor und die als Potentiostatschaltung ausgebildete Messschaltung beide im Sondenkopf der Perkutan-Messsonde integriert sind.

Die Erfindung wird nachfolgend anhand einer in den Figuren dargestellten bevorzugten Ausführungsform näher erläutert. Die dort dargestellten Besonderheiten können einzeln oder in Kombination verwendet werden, um bevorzugte Ausgestaltungen der Erfindung zu schaffen. Es zeigen:
- Fig. 1: eine schematische Ansicht der Analysevorrichtung mit einer Perkutan-Messsonde und einer Sondenanschlusseinheit;
- Fig. 2: eine Schnittzeichnung der Perkutan-Messsonde mit einem Sondenkopf und einem Lichtleiter;
- Fig. 3: eine vergrößerte Schnitt-Darstellung des Sondenkopfs aus Figur 2;
- Fig. 4: eine Schnitt-Darstellung des Sondenkopfs in einer zweiten Ebene;
- Fig. 5: die Perkutan-Messsonde aus Figur 2 mit einer Einführhilfe; und
- Fig. 6: eine Detailansicht eines Abschnitts des Lichtleiters mit Einführhilfe aus Figur 5.

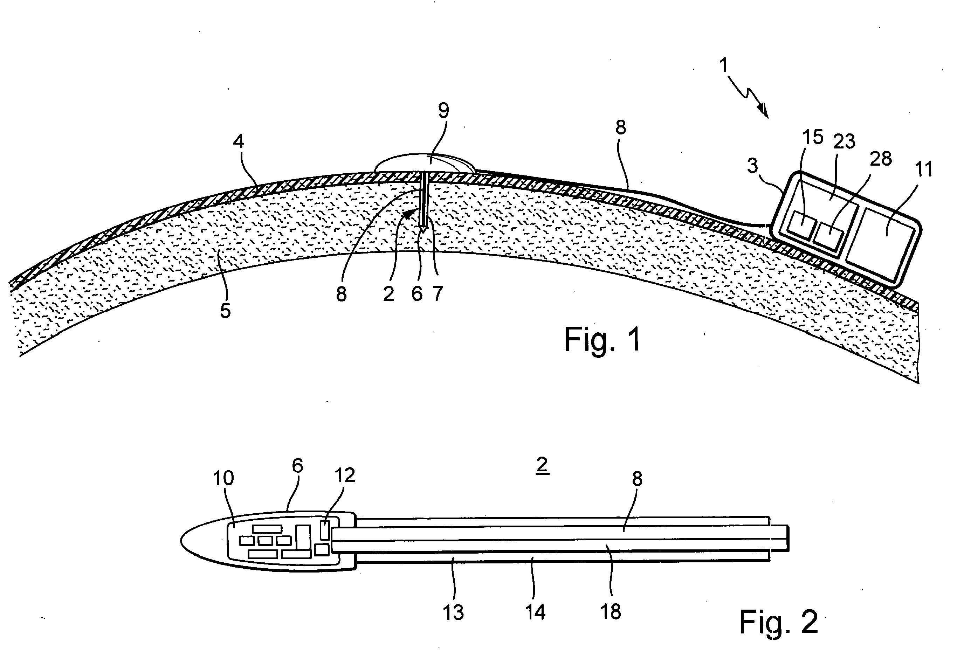

Figur 1 zeigt eine erfindungsgemäße Analysevorrichtung 1 mit einer Perkutan-Messsonde 2 und einer Sondenanschlusseinheit 3. Die Analysevorrichtung 1 wird dazu verwendet, um den Glucosegehalt eines Patienten in vivo zu bestimmen. Wenn im folgenden auf die Glucosebestimmung Bezug genommen wird, so stellt dies keine Beschränkung der Allgemeinheit dar. Die Analysevorrichtung 1 ist ebenso geeignet, um andere Analyten in Körperflüssigkeiten zu bestimmen, die sich mit einem elektrochemischen Analysesensor erfassen lassen.

Die Perkutan-Messsonde 2 ist durch die Hautoberfläche 4 hindurch eingeführt, so dass sich die Messsonde 2 in der Haut 5 eines Patienten befindet. Die Perkutan-Messsonde 2 hat einen Sondenkopf 6, in dem ein elektrochemischer Analysesensor 7 integriert ist. Der Sondenkopf 6 ist über einen Lichtleiter 8 mit der auf der Hautoberfläche 4 angeordneten Sondenanschlusseinheit 3 verbunden. Eine außen auf der Haut angeordnete Abdeckeinheit 9 dient zur Stabilisierung der Perkutan-Messsonde 2 in der Haut 5. Sie weist bevorzugt eine pilzkopfförmige Form auf, was den Tragekomfort erhöht. Die Abdeckeinheit 9 stabilisiert die Perkutan-Messsonde 2 in der Haut. Dadurch wird zum einen ein unbeabsichtigtes Entfernen der Perkutan-Messsonde 2 aus der Haut verhindert, zum anderen wird eine schmerzhafte Bewegung der Messsonde 2 in der Haut ausgeschlossen; die Messsonde 2 verbleibt in ihrer Position in der Haut.

Die Sondenanschlusseinheit 3 umfasst eine Energieversorgungseinheit 11, um die Perkutan-Messsonde 2 mit Energie zu versorgen. Dazu ist ein elektro-optischer Wandler 15 vorgesehen, um die Energie in Licht umzuwandeln, das über den Licht!eiter 8 zur Messsonde transportiert wird. In der Sondenanschlusseinheit 3 ist eine Auswerteschaltung 23 integriert, die die von der Messsonde 2 erzeugten Messsignale empfängt und auswertet, um ein analytisches Resultat zu erzeugen. Ein Lichtempfänger 28 wandelt das optisch übertragene Messsignal in ein elektrisches Messsignal um, das von der Auswerteschaltung 23 verarbeitet werden kann.

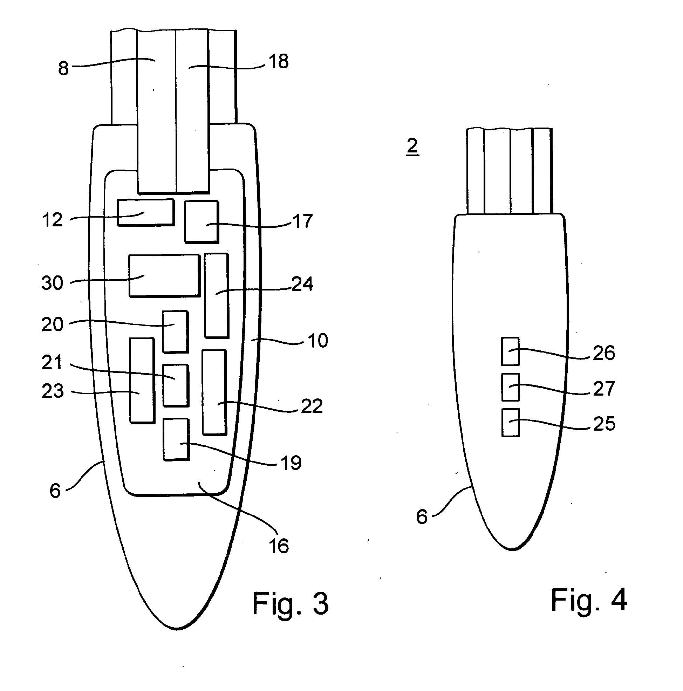

Der Lichtleiter 8, der die Messsonde 2 mit der Sondenanschlusseinheit 3 verbindet, dient zum einen zur Datenübertragung der von dem Analysesensor 7 aufgenommenen Messsignale an die in der Sondenanschlusseinheit 3 integrierte Auswerteschaltung 23. Zum anderen wird über den Lichtleiter 8 auch die Energie an die Messsonde 2 transportiert. In der Messsonde 2 ist ein optischer Empfänger 12 (Figur 3) umfasst, der das empfangene Licht wieder in elektrische Energie umwandelt und so die Sondenkopfelektronik 10 im Sondenkopf 6 mit Energie versorgt.

In einer besonders bevorzugten Ausführungsform ist der opto-elektrische Wandler der Energieversorgungseinheit 11 eine Laserdiode 15, um die Energie in Form von Licht an die Messsonde 2 zu übertragen. Anstelle der Laserdiode 15 kann in der Sondenanschlusseinheit 3 auch ein Halbleiterlaser oder ein Wechsellaser verwendet werden. Bevorzugt wird ein elektro-optischer Laser eingesetzt, der eine sehr effiziente Übertragung der Energie in Form von Licht gewährleistet. Der Einsatz von VCSEL-Lasern hat sich als besonders geeignet erwiesen. Derartige bevorzugte Laser arbeiten mit einer Wellenlänge von 700 nm und haben einen Wirkungsgrad von 70 %. Der optische Empfänger 12 in dem Sondenkopf 6 der Messsonde 2 ist dann bevorzugt als Photodiode ausgebildet, die an die Wellenlänge des VCSEL-Lasers angepasst ist und einen Wirkungsgrad von 40 % aufweist.

Figur 2 zeigt die Perkutan-Messsonde 2 mit ihrem Sondenkopf 6 und dem daran angekoppelten Lichtleiter 8. Besonders bevorzugt ist eine Ausführung, bei der neben dem Lichtleiter 8 ein weiterer Lichtleiter 18 an den Sondenkopf 6 angekoppelt. Bevorzugt dient einer der beiden Lichtleiter 8,18 der Energieübertragung an die Messsonde 2, während der andere Lichtleiter 8,18 zur Übertragung der optischen Datensignale verwendet wird. Auf diese Weise kann eine Trennung der Energieübertragung und der Datenübertragung an die Messsonde 2 realisiert werden. Es ist auch möglich, den einen Lichtleiter 8 zur Energieübertragung und gleichzeitig zur Übertragung von Datensignalen von der Sondenanschlusseinheit 3 an die Messsonde 2 zu verwenden, während der andere Lichtleiter 18 zur Datenübertragung von der Messsonde 2 an die Sondenanschlusseinheit 3 eingesetzt wird.

In einer alternativen Ausführungsform mit nur einem Lichtleiter 8 kann die Übertragung von und zu der Messsonde 2 in Form von Lichtsignalen über den einen Lichtleiter 8 durchgeführt werden. Dabei werden bevorzugt die Lichtsignale zur Datenübertragung aufmoduliert, so dass eine gleichzeitige Energieversorgung und Datenübertragung auch mit nur einem Lichtleiter 8 möglich ist.

Da die Übertragung der Analysewerte bzw. der ein analytisches Resultat charakterisierenden Messgrößen in Form von Licht stattfindet, ist eine Entkopplung und somit eine galvanische Trennung der elektrochemischen Analyseeinheit und der elektrischen Messschaltung von der Übertragung gegeben. Zusätzlich wird eine Einkopplung von elektrischen Störgrößen verhindert. Auf abschirmende Maßnahmen kann also verzichtet werden. Insbesondere kann durch geeignete Kapselung der Messsonde auch ein galvanischer Schluss und damit das Auftreten von Fehlerströmen vermieden werden.

Die in den Figuren dargestellte bevorzugte Ausführungsform zeigt die Messsonde 2 mit einer Kanüle 13, die den Lichtleiter 8 umhüllt. Vorteilhafterweise wird der Lichtleiter 8 wenigstens in dem Bereich umhüllt, der perkutan in der Haut 5 angeordnet ist.

Die Kanüle 13 ist nicht notwendigerweise eine starre Röhre. Im Sinne dieser Erfindung umfasst der Begriff "Kanüle" auch netzartige Strukturen oder Strukturen aus Gewebe, die teilweise auch verstärkt sein können. Vorzugsweise weist die Kanüle 13 eine flexible und zugfeste Hüllstruktur 14 auf. Sie besteht besonders bevorzugt aus einem Fasergewebe. Insbesondere eignen sich hierzu Kunstfasern, wobei besonders bevorzugt Synthesefasern, wie insbesondere Polyamid zum Einsatz kommen. Andere Strukturen in Form einer Kanüle sind jedoch auch denkbar, wenn diese ebenfalls die gewünschte Flexibilität und Zugfestigkeit aufweisen.

Die verwendeten Lichtleiter 8,18 bestehen bevorzugt aus einem Kunststoffmaterial, sind also "polymer optical fibres". Diese Materialien eignen sich besonders gut zur Übertragung von Licht und weisen eine geringe Dämpfung auf. Alternativ können selbstverständlich auch Glasfasern verwendet werden oder andere, vorzugsweise transparente Materialien, die zur Übertragung von Licht und für die vorgesehene Verwendung zum Anschluss an eine Perkutan-Messsonde hinreichend geeignet sind.

Die Lichtleiter 8,18 haben bevorzugt einen Durchmesser von höchstens 100 µm, vorzugsweise von höchstens 30 µm. Die in der Figur 2 gezeigten Lichtleiter 8,18 weisen einen Durchmesser von 10 µm auf, was als besonders bevorzugt angesehen wird. Durch diese dünne optische Faser ergibt sich ein hoher Gestaltungsgrad, insbesondere da sie sehr flexibel ist und sich sehr gut am Körper positionieren lässt. Aufgrund ihrer hohen Flexibilität spürt der Benutzer die Lichtleiter 8,18 im täglichen Gebrauch kaum, da sie sich an jede Bewegung des Körpers anpassen.

Die relativ dünnen Fasern mit einem Durchmesser von ca. 10 µm haben darüber hinaus den Vorteil, dass die gesamte Faser inklusive der Hüllstruktur 14 einen Gesamtdurchmesser von < 250 µm, bevorzugt von etwa 150 µm aufweist. Aufgrund dieses geringen Durchmessers ist das Schmerzempfinden des Sensors in der Haut deutlich reduziert. Die Hüllstruktur 14 kann aufgrund der sehr dünnen "polymer optical fibre" derart ausgebildet sein, dass sie sehr zugfest ist. Hierdurch wird sichergestellt, dass die Messsonde auch beim Herausziehen aus der Haut intakt bleibt und nicht beschädigt wird. Das Gewebe der Hüllstruktur 14 selbst ist sehr glatt, um ein einfaches und schmerzfreies Herausgleiten aus der Haut zu gewährleisten. Alternativ kann die Hüllstruktur auch aus mehreren entsprechend dünnen Schichten ausgebildet sein.

Figur 3 zeigt einen Schnitt durch den Sondenkopf 6 der Perkutan-Messsonde 2, an deren Sondenkopf 6 die Lichtleiter 8,18 angekoppelt sind. Im Sondenkopf 6 ist eine Platine 16 vorgesehen, die die Sondenkopfelektronik 10 aufnimmt. Die beiden Lichtleiter 8,18 sind an die Sondenkopfelektronik derart angekoppelt, dass der optische Empfänger 12, der am Ende des Lichtleiters 8 angeordnet ist, die in Form von Licht übertragene Energie in elektrische Energie umwandeln kann, um die Sondenkopfelektronik 10 zu versorgen. Ein optischer Sender 17 ist am Ende des zweiten Lichtleiters 18 angeordnet. Er wandelt elektrische Signale der Sondenkopfelektronik 10 in optische Lichtsignale um und überträgt sie durch den Lichtleiter 18 an die Sondenanschlusseinheit 3.

Die Sondenkopfelektronik 10 umfasst drei Anschlussfelder 19,20,21 zum Anschluss von Messelektroden 25,26,27, die in einer Ebene oberhalb der in Figur 3 dargestellten Schnittebene liegen. Diese Ebene ist in Figur 4 dargestellt. Eine Messschaltung 22 ist mit den Anschlussfeldern 19,20,21 der Messelektroden des Analysesensors 7 verbunden, um bei Kontakt der Messelektroden 25,26,27 mit einer Körperflüssigkeit (interstitielle Flüssigkeit, Blut) ein Messsignal zu erzeugen, das für das gewünschte analytische Glucoseresultat charakteristisch ist.

In der Ausführungsform nach Figur 3 ist die Auswerteschaltung 23 in der Sondenkopfelektronik 10 integriert. Die von der Messschaltung 22 erzeugten Messsignale werden in der Auswerteschaltung 23 ausgewertet, um eine Information über das gewünschte analytische Resultat, in diesem Fall über den Glucosegehalt des Patienten, zu gewinnen. In der Auswerteeinheit 23 ist ein Algorithmus implementiert, der hinterlegte Kalibrationsdaten verarbeitet, die in Form einer Messkurve oder einer Tabelle so vorliegen, dass eine direkte Zuordnung zwischen Messsignalen und dem Glucosegehalt gegeben ist. Die Kalibrationsdaten können aber auch von außen verändert werden, beispielsweise können geänderte Kalibrationsdaten von der Sondenanschlusseinheit 3 über die Lichtleiter 8 in die Auswerteschaltung 23 eingespeist werden. Durch Vergleich der von der Messschaltung erzeugten Messsignale wird der zugehörige Glucosegehalt bestimmt. Die ermittelte Information über den Glucosegehalt liegt in Form einer elektrischen analogen Größe vor. Diese wird in einem A/D-Wandler 24 in ein Digitalsignal umgewandelt, das dann von dem optischen Sender 17 über den Lichtleiter 18 an die Sondenanschlusseinheit 3 übertragen wird. Alternativ ist es auch möglich, die von der Messschaltung 22 erzeugten Messsignale, die in der Regel analoge Stromsignale sind, in dem A/D-Wandler 24 in eine digitale Größe zu wandeln, bevor sie der Auswerteschaltung 23 zugeführt werden. Die Auswerteschaltung 23 kann dann in Form von Software realisiert sein. Hierbei können auch einzelne Messwerte zwischengespeichert werden, z.B. um Übertragungskonflikte zu vermeiden oder um Echtzeitfunktionen zu implementieren.

Besonders bevorzugt ist die Messschaltung 22 im Sondenkopf 6 als ASIC implementiert. Darüber hinaus können auch weitere Teile der Sondenkopfelektronik 12 in dem ASIC integriert sein. Vorzugsweise ist der ASIC in Silicium-Technik realisiert. Alternativ oder zusätzlich zu der Implementierung in Silicium-Technik kann die Messschaltung 22 und/oder die Sondenkopfelektronik 10 bzw. Teile davon in Form von Polymer-Elektronik realisiert sein.

Oberhalb der Anschlussfelder 19 bis 21 sind die Messelektroden 25 bis 27 angeordnet, wie in Figur 4 zu erkennen ist. Die Messelektroden 25 bis 27 sind mit den entsprechenden Anschlussfeldern 19 bis 21 verbunden. Die Arbeitselektrode 25 umfasst ein Enzym, um die Glucosemoleküle des Patienten enzymatisch umzusetzen und das dabei entstehende Elektron als Strom zu messen. Als Enzym wird im Fall der Glucosebestimmung eine Glucoseoxidase verwendet. Zusätzlich weist die Arbeitselektrode 25 auch Mangandioxid auf. Die Messelektrode 26 wird als Gegenelektrode bezeichnet; sie ist aus Platin oder Gold hergestellt. Die Messelektrode 27 ist als Referenzelektrode aus Silber-Silberchlorid (Ag/AgCl) gefertigt.

Der gesamte Sondenkopf 6 (einschließlich der Lichtleiter) ist mit einer isolierenden Schutzhülle hermetisch abgeschlossen. Damit die Körperflüssigkeit mit den Messelektroden 25 bis 27 in Kontakt kommen kann, weist die Messsonde 2 in dieser Außenhülle Öffnungen auf, durch die die Körperflüssigkeit ein- und austreten kann. Die oberhalb der Messelektroden 25 bis 27 angeordneten Öffnungen sind mit einem (nicht dargestellten) Deckfilm verschlossen, der aus einem körperverträglichem Material gebildet ist. Der Deckfilm bildet eine permeable Membran, durch die ein diffusiver Durchtritt der Körperflüssigkeit, insbesondere der interstitiellen Flüssigkeit, möglich ist, so dass diese an die Messelektroden 25 bis 27 vordringen kann.

Die Messschaltung 22 ist als Potentiostatschaltung ausgebildet. Diese Regelschaltung regelt die Spannung zwischen der Arbeitselektrode 25 und der Gegenelektrode 26 auf einen vorgegebenen Soll-Spannungswert in Abhängigkeit von einer zwischen einer Referenzelektrode 27 und Arbeitselektrode 25 gemessenen Ist-Spannung ein. Um eine Langzeitmessung des Glucosegehalts im Blut durchzuführen, muss nach der Initialisierung die Gegenelektrode 26 permanent an einer Spannung anliegen, die im Bereich zwischen 200 mV (Millivolt) und 300 mV (Millivolt) liegt. Die hierfür benötigte Energie liegt im Nanowattsekunden- bis Mikrowattsekunden-Bereich (mWs-µWs). Hierzu ist eine permanente oder zumindest intervallmäßige Energiezuführung an die Perkutan-Messsonde 2 notwendig. Alternativ oder zusätzlich kann, um Spannungsschwankungen bzw. Schwankungen in der Energieübertragung auszugleichen, eine Pufferbatterie 30 im Sondenkopf 6 integriert sein. Diese Batterie 30 ist sehr klein und kann nur für eine gewisse, relativ kurze Zeit die Energieversorgung überbrücken, wenn kein Licht von der Energieversorgungseinheit 11 an die Sonde 2 übertragen wird. Alternativ zu der Batterie 30 könnte auch ein anderer Energiespeicher, z.B. eine Kapazität, sogenannte Super-Cabs, oder ähnliches, eingesetzt werden.

Insgesamt ist die Energieaufnahme der Messsonde 2 recht gering. Wenn die Sondenkopfelektronik 10 in einer ASIC-Struktur realisiert ist, liegt die Leistungsaufnahme bei etwa 1 µW (Mikrowatt). Wird zur Energieübertragung ein VCSEL-Laser mit 70 % Wirkungsgrad und zum Empfangen eine wellenlängenangepasste Photodiode mit 40 % Wirkungsgrad verwendet, so liegt der benötigte Energiebedarf bei ca. 10 Joule pro Tag. Mit einer herkömmlichen AAA-Batterie könnte die Messsonde 2 demnach ca. einen Monat betrieben werden.

In einer alternativen Ausgestaltung der Perkutan-Messsonde 2 kann bevorzugt ein zweiter elektrochemischer Analysesensor in dem Sondenkopf 6 integriert sein. Hiermit lässt sich ein redundantes Messsystem ausbilden, um die Messgenauigkeit und die Messsicherheit noch zu erhöhen. Alternativ kann mit dem zweiten elektrochemischen Analysesensor auch ein weiterer Analyt, beispielsweise Lactat oder ähnliches, zusätzlich zu dem Glucosewert bestimmt werden. Diese Doppelsensorstrukturen eignen sich also entweder zur verbesserten Qualitätssicherung oder zur Parallelmessung mehrerer Analyten in einer Körperflüssigkeit, so dass ein umfangreicheres Monitoring durchgeführt werden kann. In einer weiteren alternativen Ausgestaltung könnten auch zur Durchführung von Wechselstrom-Widerstandsmessungen mehrere Elektroden bzw. Hilfselektroden in der Perkutan-Messsonde 2 angeordnet sein. In einem solchen Fall wären dann vorteilhafterweise vier bis fünf weitere Elektroden vorhanden. Auch ist es möglich, zusätzlich zu dem elektrochemischen Analysesensor einen Temperaturmesser in der Messsonde 2 zu implementieren, um die Temperatur am Messort der Glucosemessung zu ermitteln. Temperaturabhängige Abweichungen könnten dann gegebenenfalls kompensiert werden.

In einer weiter bevorzugten Ausführungsform sind die Messelektroden 25 bis 27 sterilisiert, insbesondere elektronenstrahl-sterilisiert. Bevorzugt ist eine Ausführungsform, bei der der gesamte elektrochemische Analysesensor 7 elektronenstrahl-sterilisiert sind. Besonders bevorzugt ist der gesamte Sondenkopf 6 und/oder die komplette Perkutan-Messsonde 2 einschließlich der Sondenkopfelektronik 10 elektronenstrahlsterilisiert. Zu diesem Zweck findet die Sterilisierung mit Elektronenstrahlen erst nach dem Zusammenbau der Messsonde statt. Alternativ ist ein zweistufiger Herstellungsprozess durchführbar, bei dem die "Sensorchemie" (Analysesensor und Messelektroden) elektronenstrahlsterilisiert wird. Diese wird dann, nach einem Schutz gegen ein Wiederverkeimen, mit der (chemisch) sterilisierten Sondenkopfelektronik 10 gemeinsam montiert. Teilweise ist auch eine partielle Schirmung der Sondenkopfelektronik 10 während der Elektronenbestrahlung (e-beam) zur Sterilisierung möglich.

Wenn nicht die komplette, sondern nur Teile der Auswerteschaltung 23 in der Sondenkopfelektronik 10 integriert sind, werden aus den von der Messschaltung 22 erzeugten Messsignalen Zwischenergebnisse ermittelt, die über die Lichtleiter 8,18 an die Sondenanschlusseinheit 3 übertragen werden. Die Sondenanschlusseinheit 3 umfasst dann einen weiteren Teil der Auswerteschaltung 23, um die übertragenen Zwischenergebnisse auszuwerten und/oder aufzubereiten. Die Sondenanschlusseinheit 3 kann darüber hinaus noch eine Anzeige einschließen, um den Analysewert, insbesondere den Glucosegehalt auszugeben. Darüber hinaus kann auch ein Funksender in der Sondenanschlusseinheit 3 integriert sein, um die Werte an eine externe Einheit zu übertragen. In der externen Einheit können die Analysewerte entweder nur angezeigt oder auch aufbereitet und archiviert werden. Die Übertragung von der Sondenanschlusseinheit 3 an die externe Einheit kann über gängige Funkübertragungen stattfinden, beispielsweise über Infrarot oder Bluetooth oder Wireless-Lan-Verbindungen.

Alternativ kann auch ein Teil der Auswerteschaltung 23 in der Abdeckeinheit 9 integriert sein. Die Abdeckeinheit 9 kann dann ebenfalls über ein Funkmodul verfügen, um die Analyseergebnisse an eine externe Einheit zu übertragen. Die Sondenanschlusseinheit 3 umfasst in diesem Fall lediglich die Energieversorgungseinheit 11 inklusive der notwendigen elektro-optischen Wandler. Darüber hinaus kann auch die komplette Sondenanschlusseinheit 23 in der Abdeckeinheit 9 integriert sein.

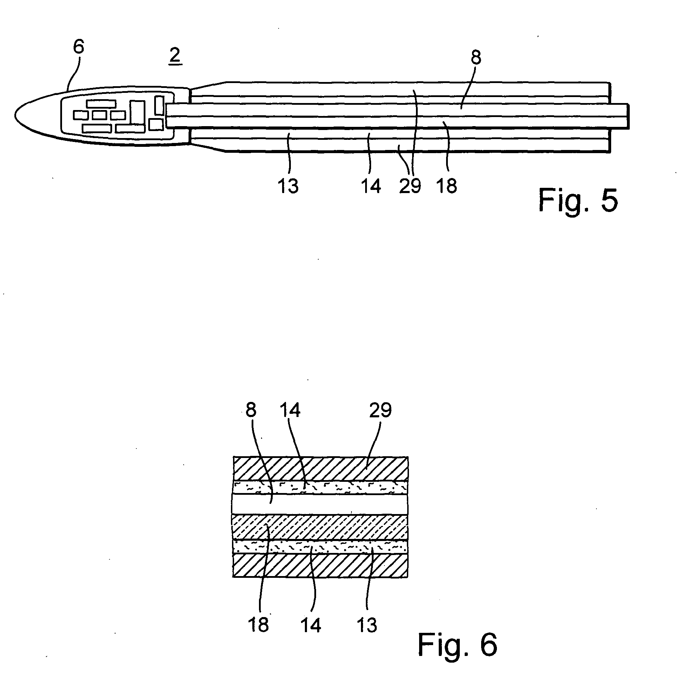

Um den Sondenkopf 6 der Perkutan-Messsonde 2 durch die Haut in den Körper des Patienten einzuführen, kann eine Einführhilfe 29 verwendet werden, die beispielsweise als Kanüle, Rohr oder Metallrohr ausgebildet sein kann. Dabei eignen sich insbesondere Kanülen mit einem Längsschlitz, so dass die Einführhilfe 29 nach Plazieren der Perkutan-Messsonde 2 in der Haut einfach wieder entfernt werden kann. Die Lichtleiter 8,18 können durch den offenen Schlitz der Einführhilfe 29 herausgleiten. Figur 5 zeigt die Perkutan-Messsonde 2, die in der Einführhilfe 29 positioniert ist. Der Sondenkopf 6 ragt über die Einführhilfe 29 heraus; die Lichtleiter 8,18 und die Hüllstruktur 14 werden durch die Einführhilfe 29 stabilisiert.

Eine Detailansicht eines Teils der Lichtleiter 8,18 mit Einführhilfe 29 ist in Figur 6 gezeigt. Die als Metallrohr ausgeführte Einführhilfe 29 umschließt die Hüllstruktur 14 und die beiden Lichtleiter 8,18 zur Energie- bzw. Datenübertragung von bzw. zu der Perkutan-Messsonde 2. Deutlich zu erkennen ist die gewebeartige Struktur der Kanüle 13, die eine flexible aber sehr zugfeste Stützung und einen entsprechenden Schutz-der Lichtleiter gewährleistet.

## Patentansprüche

1. Analysevorrichtung zur Bestimmung eines Analyten in vivo im Körper eines Patienten, umfassend
eine zum Einführen durch die Hautoberfläche (4) in den Körper ausgebildete Perkutan-Messsonde (2) mit einem Sondenkopf (6), der einen elektrochemischen Analysesensor (7) mit zwei Messelektroden (25,26,27) aufweist,
eine am Körper tragbare Sondenanschlusseinheit (3) zum Anschließen an die Perkutan-Messsonde (2),
eine mit den Messelektroden (25,26,27) verbundene Messschaltung (22), wobei die Messschaltung (22) bei Kontakt der Messelektroden (25,26,27) mit einer Körperflüssigkeit ein Messsignal erzeugt, das für das gewünschte analytische Resultat charakteristisch ist,
eine Auswerteschaltung (23), um Messsignale aus der Messschaltung (22) auszuwerten und eine Information über das gewünschte analytische Resultat zu gewinnen,
**dadurch gekennzeichnet, dass**
die Messschaltung (22) als Bestandteil einer Sondenkopfelektronik (10) in dem Sondenkopf (6) integriert ist,
der Sondenkopf (6) an einen Lichtleiter (8,18) angekoppelt ist,
der Sondenkopf (6) einen optischen Sender (17) aufweist, um elektrische Signale der Sondenkopfelektronik (10) in Lichtsignale umzuwandeln und um die Lichtsignale durch den an dem Sondenkopf (6) angekoppelten Lichtleiter (8,18) zu übertragen, und
die Sondenanschlusseinheit (3) einen Lichtempfänger (28) enthält, um die Lichtsignale von der Messsonde (2) zur Weiterverarbeitung zu empfangen.

2. Analysevorrichtung nach Anspruch 1, **dadurch gekennzeichnet, dass** die Messschaltung (22) eine Potentiostatschaltungsanordnung ist, durch die die Spannung zwischen den Messelektroden (25,26,27) eingestellt wird.

3. Analysevorrichtung nach Anspruch 2, **dadurch gekennzeichnet, dass** der Analysesensor (7) eine zusätzliche Referenzelektrode (27) enthält.

4. Analysevorrichtung nach einem der vorhergehenden Ansprüche, **dadurch gekennzeichnet, dass** die Messsonde (2) eine Kanüle (13) umfasst, die den Lichtleiter (8) wenigstens in dem Bereich umhüllt, der perkutan im Körper anordenbar ist.

5. Analysevorrichtung nach Anspruch 4, **dadurch gekennzeichnet, dass** die Kanüle (13) eine flexible und zugfeste Hüllstruktur (14) bevorzugt mit einem Fasergewebe aufweist.

6. Analysevorrichtung nach einem der vorhergehenden Ansprüche, **dadurch gekennzeichnet, dass** eine Energieversorgungseinheit (11), die vorzugsweise in der Sondenanschlusseinheit (3) integriert ist, am Körper tragbar ist und Licht von der Energieversorgungseinheit (11) über den Lichtleiter (8,18) an die Messsonde (2) transportiert wird, das von einem in der Messsonde (2) angeordneten optischen Empfänger (12) in elektrische Energie umgewandelt wird.

7. Analysevorrichtung nach Anspruch 6, **dadurch gekennzeichnet, dass** die Übertragung von und zu der Messsonde (2) in Form von Lichtsignalen über den Lichtleiter (8,18) durchgeführt wird, wobei die Lichtsignale zur Datenübertragung aufmoduliert werden.

8. Analysevorrichtung nach einem der vorhergehenden Ansprüche, **gekennzeichnet durch** zwei Lichtleiter (8,18), wobei ein erster Lichtleiter (8) der Energieübertragung an die Messsonde (2) dient und ein zweiter Lichtleiter (18) optische Datensignale überträgt.

9. Analysevorrichtung nach einem der Ansprüche 6 bis 8, **dadurch gekennzeichnet, dass** die Energieversorgungseinheit (11) eine Laserdiode (15) aufweist, die vorzugsweise ein VCSEL-Laser ist.

10. Analysevorrichtung nach Anspruch 6, **dadurch gekennzeichnet, dass** der optische Empfänger (12) in dem Sondenkopf (6) der Messsonde (2) eine Photodiode ist.

11. Analysevorrichtung nach einem der vorhergehenden Ansprüche, **dadurch gekennzeichnet, dass** der Sondenkopf (6) der Messsonde (2) einen A/D-Wandler (24) umfasst, um die analogen elektrischen Signale der Sondenkopfelektronik (10) in digitale Signale umzuwandeln, die von der Messsonde (2) zu der Sondenanschlusseinheit (3) über den Lichtleiter (8,18) übertragen werden.

12. Analysevorrichtung nach einem der vorhergehenden Ansprüche, **dadurch gekennzeichnet, dass** der Lichtleiter (8,18) aus einem Kunststoffmaterial besteht.

13. Analysevorrichtung nach einem der vorhergehenden Ansprüche, **dadurch gekennzeichnet, dass** der Lichtleiter (8,18) einen Durchmesser von höchstens 100 µm, vorzugsweise von höchstens 30 µm, besonders bevorzugt von höchstens 10 µm hat.

14. Analysevorrichtung nach einem der vorhergehenden Ansprüche, **dadurch gekennzeichnet, dass** die Messschaltung (22), bevorzugt die Sondenkopfelektronik (10), im Sondenkopf (6) als ASIC implementiert ist, vorzugsweise in Siliciumtechnik.

15. Analysevorrichtung nach einem der vorhergehenden Ansprüche, **dadurch gekennzeichnet, dass** der Sondenkopf (6) einen zweiten elektrochemischen Analysesensor umfasst.

16. Analysevorrichtung nach einem der vorhergehenden Ansprüche, **dadurch gekennzeichnet, dass** die Messelektroden (25,26,27), bevorzugt der elektrochemische Analysesensor (7), besonders bevorzugt der Sondenkopf (6) der Messsonde (2) elektronenstrahl-sterilisiert sind.

17. Analysevorrichtung nach einem der vorhergehenden Ansprüche, **dadurch gekennzeichnet, dass** die Messsonde (2) eine an der Hautoberfläche (4) anliegende Abdeckeinheit (9) umfasst, um den Sondenkopf (6) und/oder Lichtleiter (8,18) zu stabilisieren.

18. Analysevorrichtung nach Anspruch 17, **dadurch gekennzeichnet, dass** die Abdeckeinheit (9) derart ausgebildet ist, dass Teile der Auswerteschaltung (23) in der Abdeckeinheit (9) integriert sind.
